(19) Europäisches Patentamt
European Patent Office
Office européen des brevets

(11) EP 1 097 721 B1

(12) EUROPEAN PATENT SPECIFICATION

(45) Date of publication and mention of the grant of the patent:
**14.05.2003 Bulletin 2003/20**

(51) Int Cl.[7]: **A61K 9/113**

(21) Application number: **00123909.4**

(22) Date of filing: **03.11.2000**

# (54) Oil-based adjuvant vaccine

Öladjuvierter Impfstoff

Adjuvant pour vaccin à base d'huile

(84) Designated Contracting States:
**BE DE DK ES FR GB IT NL**

(30) Priority: **05.11.1999 JP 31612199**

(43) Date of publication of application:
**09.05.2001 Bulletin 2001/19**

(73) Proprietors:
- **NOF CORPORATION**
  **Tokyo 150-6019 (JP)**
- **Juridical Foundation,**
  **The Chemo-Sero-Therapeutic Research Institute**
  **Kumamoto-shi, Kumamoto 860-8568 (JP)**

(72) Inventors:
- **Saito, Koichi**
  **Amagasaki-shi, Hyogo 661-0012 (JP)**
- **Kishimoto, Yoko**
  **Akashi-shi, Hyogo 674-0081 (JP)**
- **Miyahara, Tokuji**
  **Kikuchi-gun, Kumamoto 861-1112 (JP)**
- **Takase, Kouzou**
  **Kikuchi-gun, Kumamoto 869-1236 (JP)**

(74) Representative:
**von Kreisler, Alek, Dipl.-Chem. et al**
**Patentanwälte,**
**von Kreisler-Selting-Werner,**
**Bahnhofsvorplatz 1 (Deichmannhaus)**
**50667 Köln (DE)**

(56) References cited:
- **DATABASE CHEMABS [Online] CHEMICAL ABSTRACTS SERVICE, COLUMBUS, OHIO, US; MIYANOHARA, TOKUJI ET AL: "Oil adjuvant vaccine" retrieved from STN Database accession no. 130:316623 XP002163309 & JP 11 106351 A (CHEMO-SERO-THERAPEUTIC RESEARCH INSTITUTE, JAPAN;NIPPON OIL AND FATS C) 20 April 1999 (1999-04-20)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).



Printed by Jouve, 75001 PARIS (FR)

## Description

### FIELD OF THE INVENTION

The present invention relates to an oil adjuvant vaccine. More particularly, the present invention relates to a W/O/W type oil adjuvant vaccine superior in an adjuvant effect and stability as a preparation.

### BACKGROUND OF THE INVENTION

Oil adjuvant vaccines have long been known to efficiently potentiate immunity. Particularly, the Freund's adjuvant shows an extremely superior immunity potentiating effect when using an inactivated antigen and is a representative adjuvant widely used in animal tests. However, since this adjuvant also causes strong side effects such as severe abscess and granuloma, it is used only in laboratories.

Thus, many attempts have been made to reduce such side effects and to apply an oil adjuvant to general use. Oil adjuvants have various preparation types, such as a W/O type like the Freund's adjuvant, an O/W type superior in safety, a complex W/O/W type and the like.

For example, JP-B-6-81731 reports a W/O type oil adjuvant containing anhydrous mannitol oleic ester as a surfactant and liquid paraffin as an oil component, and WO91/00107 (Japanese Patent Application under PCT laid-open under Kohyo No. 4-506521) reports a low viscosity W/O type oil adjuvant containing a metabolizable oil, such as vegetable oil, and an unmetabolizable oil, such as mineral oil in combination as an oil component.

In addition, WO90/14837 discloses an O/W type adjuvant as an adjuvant preparation containing a sub-micron oil drop emulsion, and EP-A-315153 discloses an O/W type vaccine adjuvant containing an emulsifier containing a POP-POE block polymer and glycopeptide as an immunostimulant.

Of these adjuvants, a W/O type oil adjuvant shows a high immunopotentiating effect but has a tendency to remain at the site of injection due to the continuous oil phase of the preparation. In not rare instances, it develops sterile abscess and granuloma in the vicinity of an inoculation site, and possibly causes a sharp pain during inoculation. Therefore, this type of oil adjuvant has been limited in use as a preparation allowing for direct inoculation to a body. An O/W type oil adjuvant shows a light topical response at an injection site and is superior in safety. However, since an antigen is contained in a continuous phase, it quickly diffuses at the site of injection, making a sufficient effect difficult to be provided, and often requires a specific immunostimulant.

In view of the above situation, a new type of an oil adjuvant, W/O/W type oil adjuvant, has been under investigation in recent years.

For example, WO91/00106 discloses a W/O/W type multi-phase emulsion that becomes W/O type upon exposure to the body temperature of an animal after injection. This technique fails to solve all the problems associated with conventional W/O type oil adjuvants, such as topical response and the like, because an oil adjuvant is present in the body in the form of a W/O type adjuvant.

Because a W/O/W type oil adjuvant takes the form of a complex emulsion, the stability as a preparation cannot be maintained easily, as compared to a W/O type and an O/W type oil adjuvants. Therefore, a W/O/W type oil adjuvant vaccine expected to simultaneously show the characteristic features of a W/O type that exhibits superior adjuvancy and an O/W type superior in safety poses many problems to solve before it enables development of an oil adjuvant capable of exhibiting well-balanced characteristics of the two types.

The present inventors have shown, in USP 5,814,321, a W/O type oil adjuvant vaccine superior in safety and preparation stability and a W/O/W type oil adjuvant vaccine obtained by dispersing the W/O vaccine in an aqueous phase.

JP-A-11-106351 discloses an oil adjuvant vaccine containing A (10-90% by weight of an oil ingredient that occurs as a liquid at ordinary temperature), B (0.5-30% by weight of an emulsifier agent selected from among non-ionic surfactants), and C (an aqueous ingredient containing 0.1-30% by weight of polyethylene glycol and a biologically acceptable effective amount of antigen).

However, a W/O/W type oil adjuvant vaccine prepared by a conventional technique shows great variation in properties depending on the formulation and the method of preparation. It is associated with the problems in that an antigen component sometimes increases viscosity strikingly and stable introduction of immunity is not feasible as compared to a W/O type oil adjuvant.

### SUMMARY OF THE INVENTION

It is therefore an object of the present invention to provide a W/O/W type oil adjuvant vaccine improved in storage stability as a preparation and in topical response at an injection site, which shows a high immunological effect.

In accordance with the present invention, there has been provided a W/O/W type oil adjuvant vaccine comprising, in an outer aqueous phase, a specific amount of a polyethylene glycol derivative having a specific molecular weight, which vaccine shows high stability as a preparation, superior safety in terms of topical response and the like, and a high immunopotentiating effect.

Accordingly, the present invention provides the following.

(1) A W/O/W type oil adjuvant vaccine comprising an outer aqueous phase containing 0.5 - 20 wt% of a polyethylene glycol derivative having a molecular weight of 400 - 20,000, which is represented by the following formula (I)

$$R^1-O-(CH_2-CH_2-O)_n-R^2 \qquad (I)$$

wherein $R^1$ and $R^2$ may be the same or different and each is a hydrogen atom or alkyl having 1 to 4 carbon atoms and n is a polymerization degree, and an inner aqueous phase containing a biologically acceptable and effective amount of an antigen.

(2) The oil adjuvant vaccine of the above-mentioned (1), wherein the polyethylene glycol derivative of the formula (I) has a molecular weight of 1,000 - 10,000.

(3) The oil adjuvant vaccine of the above-mentioned (1), wherein the outer aqueous phase contains 1 - 10 wt% of the polyethylene glycol derivative of the formula (I).

(4) The oil adjuvant vaccine of the above-mentioned (1), which is a W/O/W type oil adjuvant vaccine prepared by dispersing or emulsifying a W/O emulsion prepared from an oil component (A) which becomes liquid at room temperature, an emulsifier (B) and an aqueous component (C) containing a biologically acceptable and effective amount of an antigen, in a liquid containing an emulsifier (D) and an aqueous component (E), wherein the liquid contains 0.5 - 20 wt% of the polyethylene glycol derivative of the formula (I) having a molecular weight of 400 - 20,000.

(5) The oil adjuvant vaccine of the above-mentioned (1), which is a W/O/W type oil adjuvant vaccine prepared by dispersing or emulsifying a W/O emulsion prepared from an oil component (A) which becomes liquid at room temperature, an emulsifier (B) and an aqueous component (C) containing a biologically acceptable and effective amount of an antigen, in a liquid containing an emulsifier (D) and an aqueous component (E), and then adding the polyethylene glycol derivative of the formula (I), which has a molecular weight of 400 - 20,000, to the outer aqueous phase to a concentration of 0.5 - 20 wt%.

(6) The oil adjuvant vaccine of the above-mentioned (4) or (5), wherein the oil component (A), which becomes liquid at room temperature, contains a fatty acid ester and/or squalene in a proportion of not less than 20 wt% of an oil phase.

(7) The oil adjuvant vaccine of the above-mentioned (4) or (5), wherein the emulsifier (B) has an HLB of less than 10.

(8) The oil adjuvant vaccine of the above-mentioned (7), wherein the emulsifier (B) contains at least one member selected from a partial ester of polyhydric alcohol and a fatty acid and a non-ionic surfactant having a polyoxyethylene chain.

(9) The oil adjuvant vaccine of the above-mentioned (4) or (5), wherein the emulsifier (D) has an HLB of not less than 10.

(10) The oil adjuvant vaccine of the above-mentioned (9), wherein the emulsifier (D) contains a non-ionic surfactant having a polyoxyethylene chain.

## DETAILED DESCRIPTION OF THE INVENTION

The oil adjuvant vaccine of the present invention is prepared by dispersing or emulsifying (Step 2) a W/O emulsion prepared (Step 1) from an oil component (A) which becomes liquid at room temperature, an emulsifier (B) and an aqueous component (C) containing a biologically acceptable and effective amount of an antigen, in a liquid containing an emulsifier (D) and an aqueous component (E), wherein an outer aqueous phase of the ultimately obtained W/O/W type oil adjuvant vaccine contains a specific amount of a polyethylene glycol derivative having a specific molecular weight.

The oil component (A) which becomes liquid at room temperature constitutes a continuous phase of the W/O emulsion prepared in Step 1, and constitutes an oil phase that becomes a dispersion phase of a W/O/W type oil adjuvant vaccine obtained in Step 2. In the present invention, by the oil phase is meant an oil phase in this W/O/W type oil adjuvant vaccine. In this specification, by "at room temperature" is meant a temperature in the range of 15 - 25°C. The oil component (A) which becomes liquid at room temperature can be selected for use from ester and non-ester oil bases conventionally used for food, pharmaceutical products, cosmetics and the like, which become liquid at room temperature.

The non-ester oil base which becomes liquid at room temperature may be, for example, hydrocarbon such as light liquid paraffin, squalene, squalane, polybutene and the like, fatty acid such as saturated middle chain fatty acid (e.g., caprylic acid, capric acid and the like), long chain unsaturated fatty acid (e.g., oleic acid, linolic acid, linolenic acid and the like), middle chain or long chain aliphatic alcohol and the like. Examples of the ester oil base which is liquid at room temperature include various fatty acid esters derived from saturated middle chain fatty acid such as caprylic acid and capric acid, or unsaturated long chain fatty acid such as oleic acid and linolic acid and alcohol, as well as naturally occurring fatty acid esters, such as liquid vegetable oil (e.g., peanut oil, olive oil, safflower oil, sunflower oil, jojoba oil and the like), a liquid oil from animals, such as orange roughy oil, and the like.

As the oil component (A), one of the above examples may be used, or two or more from the above examples may be mixed and used. Of these oil components, an oil such as fatty acid esters, vegetable oil and squalene beneficially have relatively high resistance to oxidation and high affinity for a body. As an oil component to be used for the oil adjuvant vaccine of the present invention, therefore, one or more from these oils is/are preferably selected. Particularly, as a fatty acid ester, an oil base made from an esterified compound of cis-$\Delta$9-unsaturated fatty acid having 16 to 22 carbon atoms, which is also a constituent component of a biological lipid, and alcohol having 1 to 3 carbon atoms, is preferable.

Moreover, by containing a fatty acid ester and/or squalene in an oil phase at least in a proportion of not less than 20 wt%, the final oil adjuvant vaccine can further reduce side effects such as topical response and the like and improve safety. When a fatty acid ester and squalene are concurrently used, the total amount thereof should be at least not less than 20 wt% of the oil phase.

In the present invention, one or more surfactants showing high safety for a body, which are generally used for pharmaceutical products and food, can be selected and used as the emulsifier (B) and the emulsifier (D) constituting an oil adjuvant vaccine.

The emulsifier (B) is used in Step 1 for preparation of a W/O emulsion consisting of an aqueous component (C) containing an antigen and an oil component (A).

A surfactant used as the emulsifier (B), in view of the stability of the final W/O/W type oil adjuvant vaccine, is preferably lipophilic. That is, it preferably has a relatively low HLB (Hydrophilic-Lipophile Balance), particularly preferably HLB of less than 10. The emulsifier (B) may be of a single kind or a combination of two or more kinds. In this case, the emulsifier (B) is preferably selected to make the overall HLB less than 10.

More specifically, a partial ester of polyhydric alcohol and fatty acid, such as sorbitan fatty acid ester (e.g., sorbitan monooleate, sorbitan dioleate, sorbitan sesquioleate etc.), glycerol fatty acid ester (e.g., glycerol monooleate etc.), polyglycerol fatty acid ester (e.g., diglycerol monooleate, diglycerol dioleate etc.) and the like, a non-ionic surfactant, having a polyoxyethylene chain in a molecule, such as polyoxyethylene sorbitan fatty acid ester (polysorbate) [e.g., polyoxyethylene(20)sorbitan monooleate etc.], polyoxyethylene hydrogenated castor oil, polyoxyethylene polyoxypropylene glycol and the like are used. Glycerophospholipid containing naturally occurring lecithin can be also used.

Of these, a partial ester of polyhydric alcohol having 3 to 10 hydroxyl groups in a molecule, such as sorbitan, glycerol and polyglycerol, and a fatty acid having 12 to 20 carbon atoms, and hydroxy fatty acid triglyceride added with a polyoxyethylene chain, which is represented by polyoxyethylene hydrogenated castor oil, are particularly used in combination, such that the overall HLB of the emulsifier (B) is less than 10. In this way, a W/O/W type oil adjuvant vaccine having much superior stability as a preparation can be obtained.

In the present specification, the HLB is determined according to the formula proposed by W.C. Griffin [W.C. Griffin, J. Soc. Cosmetic. Chemists, 1, 311(1949)].

For the preparation of the oil adjuvant vaccine of the present invention, an emulsifier composition is used as a component constituting a W/O emulsion containing an antigen solution, which is obtained by mixing, in advance, amino acid or a salt thereof and an aqueous solution containing nonreducing sugar or sugar alcohol having at least 5 hydroxyl groups in a molecule, and partial ester of polyhydric alcohol having 3 or more hydroxyl groups and a fatty acid, which is a non-ionic surfactant that becomes liquid at room temperature, along with the above-mentioned emulsifier (B) to enhance the effectiveness of the oil adjuvant and preparation stability. The amino acid or a salt thereof, and sugar or sugar alcohol used in the form of an aqueous solution can be selected from those generally used for food, pharmaceutical products listed in the Japanese Pharmacopoeia and Japanese Pharmaceutical Codex drugs. The antigen solution used here may have any form as long as it is a liquid, and is exemplified by solution, suspension and the like. For example, the amino acid or a salt thereof may be glycine, alanine, arginine hydrochloride, histidine, phenylalanine, sodium aspartate, potassium aspartate, sodium glutamate, potassium glutamate or a hydrate thereof, wherein sugar and sugar alcohol are preferably selected from trehalose, xylitol, sorbitol, mannitol, maltitol, lactitol and the like.

The above-mentioned emulsifier composition is obtained by preparing an aqueous solution containing the above-mentioned amino acid or a salt thereof and the above-mentioned sugar or sugar alcohol, and mixing and stirring the aqueous solution with the above-mentioned non-ionic surfactant at a weight ratio of 10:1- 1:1 (aqueous solution: surfactant). The emulsifier composition is used in a proportion of about 1 - 20 wt%, preferably about 2 - 10 wt%, of a

W/O emulsion containing an antigen solution.

The emulsifier (D) is used in Step 2 for dispersing or emulsifying the above-mentioned W/O emulsion in a liquid containing an emulsifier (D) and an aqueous component (E).

A surfactant used as emulsifier (D), in view of the stability of the final W/O/W type oil adjuvant vaccine, is preferably hydrophilic and has a relatively high HLB value, which is particularly preferably not less than 10. This emulsifier (D) can be used alone or in combination on demand. In this case, the emulsifier (D) is preferably determined to make the overall HLB not less than 10.

More specifically, a non-ionic surfactant having a polyoxyethylene chain in a molecule and having an HLB of not less than 10, such as polyoxyethylene sorbitan fatty acid ester(s), polyoxyethylene hydrogenated castor oil, polyoxyethylene polyoxypropylene glycol and the like, is used. These surfactants may be used in combination with a partial ester of polyhydric alcohol and fatty acid, such as sorbitan fatty acid ester, glycerol fatty acid ester, sucrose fatty acid ester, polyglycerol fatty acid ester and the like, and glycerophospholipid containing naturally occurring lecithin. Of these, the use of particularly polyoxyethylene polyoxypropylene glycol alone or in combination with other surfactants enables preparation of a W/O/W type oil adjuvant vaccine having superior preparation stability.

The glycerophospholipid to be used as emulsifier (B) or emulsifier (D) may be, for example, naturally occurring phospholipid such as phospholipid extracted and purified from egg yolk and soy beans and hydrogenated adduct thereof, and various synthetic phospholipids and lysophospholipids wherein the composition of acyl moiety and base moiety has been controlled.

In the W/O/W type oil adjuvant vaccine of the present invention, the aqueous component (C) constitutes an aqueous phase which is a dispersion phase in the W/O emulsion prepared in Step 1 and constitutes an inner aqueous phase dispersed in an oil phase that is a dispersion phase in the final W/O/W type oil adjuvant vaccine. The aqueous component (C) contains various antigens.

The antigen to be contained in the aqueous component (C) may be of various kinds and may have various forms. More specifically, inactivated cells, inactivated viral particles, inactivated Mycoplasma generally used, as well as pathogen infection preventive factor, such as attachment protein, envelope antigen and the like used for subunit vaccine, are used. The inactivated cells may be Gram-negative bacteria such as *Actinobacillus pleuropneumoniae, Escherichia coli*, etc., Gram-positive bacteria such as *Erysipelothrix rhusiopathiae*, etc., and the like. The virus of the inactivated viral particle includes, for example, Japanese encephalitis virus and the like. The Mycoplasma of the inactivated Mycoplasma is exemplified by Mycoplasma of pig and chicken. As the pathogen infection preventive factor, F protein of New castle disease virus is exemplified. It is also possible to use a combined vaccine containing plural antigens.

The oil adjuvant vaccine of the present invention may contain, in addition to an antigen, an efficacious component other than the antigen, such as antibiotics. Examples of the antibiotics include Kanamycin sulfate, oxytetracycline hydrochloride and the like.

The biologically acceptable and effective amount of an antigen in aqueous component (C) is determined as appropriate according to the object and the kind of antigen to be used.

In the present invention, the above-mentioned antigen is contained in the inner aqueous phase. Even when a part of the antigen leaks out into the outer aqueous phase during the preparation process of the W/O/W type oil adjuvant vaccine of the present invention, the effect of the present invention is not impaired.

In the present invention, the aqueous component (E) constitutes the outer aqueous phase of the W/O/W type oil adjuvant vaccine. More specifically, the aqueous component (E) is selected as appropriate in consideration of the stability and safety of the final adjuvant vaccine to the body. For example, physiological saline, phosphate buffered physiological saline and the like are used.

According to the present invention, the polyethylene glycol derivative contained in the outer aqueous phase of an oil adjuvant vaccine is expressed by the following formula (I):

$$R^1-O-(CH_2-CH_2-O)_n-R^2 \qquad \text{(I)}$$

wherein $R^1$ and $R^2$ may be the same or different and each is a hydrogen atom or alkyl having 1 to 4 carbon atoms and n is a polymerization degree.

In the above-mentioned formula (I), alkyl having 1 to 4 carbon atoms at $R^1$ and $R^2$ may be a linear chain or branched chain, and is exemplified by methyl, ethyl, propyl, isopropyl, n-butyl, isobutyl, sec-butyl and tert-butyl. Suitable $R^1$ and $R^2$ are hydrogen atom, methyl and ethyl, wherein hydrogen atom is particularly suitable. Suitable combinations of $R^1$ and $R^2$ are hydrogen atom and hydrogen atom, hydrogen atom and methyl, hydrogen atom and ethyl, methyl

and methyl and the like. The combination of hydrogen atom and hydrogen atom is particularly suitable. The n is a polymerization degree that makes the molecular weight within the range of 400 - 20,000.

The polyethylene glycol derivative of the above-mentioned formula (I) has a molecular weight of 400 - 20,000, preferably 1,000 - 10,000, particularly preferably 3,000 - 9,000. It is contained in the outer aqueous phase in a proportion of 0.5 - 20 wt%, preferably 1 - 10 wt%, more preferably 1 - 5 wt%, of said phase.

When the polyethylene glycol derivative has a molecular weight of less than 400 or when the content is less than 0.5 wt%, a sufficient viscosity-decreasing effect cannot be achieved. In addition, the effect as an adjuvant may be weakened. When the molecular weight of the polyethylene glycol derivative exceeds 20,000, the outer aqueous phase itself has an increased viscosity, which in turn increases the viscosity of the oil adjuvant vaccine as a whole, thereby providing an insufficient viscosity-decreasing effect. When the content exceeds 20 wt%, the inner aqueous phase and the outer aqueous phase show completely different osmotic pressures. As a result, a stable W/O/W type emulsion cannot be afforded, which in turn possibly makes preparation of a stable oil adjuvant vaccine unattainable.

In the present invention, a higher molecular weight of the above-mentioned polyethylene glycol derivative causes a higher viscosity of the outer aqueous phase, as a result of which the oil adjuvant vaccine has a higher viscosity. It is desirable to adjust its content to fall within the above-mentioned range according to the molecular weight of the polyethylene glycol derivative.

According to the present invention, a polyethylene glycol derivative, from among those exemplified above having a molecular weight in the range of 400 - 20,000, may be used alone or two or more derivatives having different molecular weights may be used in combination.

In view of the stability as a preparation and an adjuvant effect of the final oil adjuvant vaccine, a polyethylene glycol derivative preferably has a molecular weight of 1000 - 10,000, and is contained in an outer aqueous phase particularly in a proportion of 1 - 10 wt%, preferably 1 - 5 wt%, of said phase.

The oil adjuvant vaccine prepared according to the present invention is characterized by superior adjuvant effect and lower viscosity that are attributable to the fact that the polyethylene glycol derivative is present in the outer aqueous phase. In general, a W/O/W type emulsion often shows a highly increased viscosity and aggregation of W/O particles, due to the effect of the substances contained in the inner aqueous phase and to the fact that different components are contained in the inner aqueous phase and the outer aqueous phase. Particularly, an antigen solution used for an oil adjuvant vaccine could contain various ingredients at high concentrations, such as cell components and medium components. For use as a W/O/W type oil adjuvant vaccine, therefore, W/O/W type emulsions having the same composition may show too dramatically different levels of viscosity and stability, which is due to the effect of other components in the antigen solution to be used. This poses a serious problem particularly when plural kinds of antigens are admixed to give a vaccine, and needs to be avoided by all means during the production and control of a vaccine. In the present invention, however, a polyethylene glycol derivative is contained in the outer aqueous phase to reduce viscosity, irrespective of composition or components of the inner aqueous phase, which in turn overcomes such problem. When an oil adjuvant vaccine has a high viscosity, the workability during administration to a body becomes poor, which may in turn result in a lower diffusing performance in the body and adverse effects, such as topical response, and residual vaccine at an injection site. By the addition of a polyethylene glycol derivative to the outer aqueous phase, these aspects can be also improved.

One of the major factors that affect the properties of the W/O/W type oil adjuvant is the particle size of the W/O emulsion contained in the outer aqueous phase. In general terms, when the particle size of the W/O emulsion contained in the outer aqueous phase is large, the W/O/W type oil adjuvant shows a behavior of a W/O type oil adjuvant upon inoculation. This means that the adjuvant effect becomes higher but the side effects, such as topical response, also become strong. In addition, particles tend to be agglomerated, thereby lowering the stability as a preparation. In contrast, when a W/O emulsion contains relatively smaller particles, the preparation has a higher stability, reduced side effects and a weaker adjuvant effect. The W/O/W type oil adjuvant vaccine prepared by the present invention shows a high immunopotentiating effect even when W/O emulsion particles are relatively small. Thus, the inventive vaccine shows superior stability as a preparation, fewer and lower levels of side effects and superior effectiveness. From the aspects of the stability as a preparation and reduction of the side effects, substantially all of the W/O emulsion particles constituting the W/O/W type oil adjuvant vaccine preferably have a particle size of not more than 50 μm and an average particle size of not more than 20 μm.

The production method of the oil adjuvant vaccine of the present invention is not particularly limited and can be a known method. When the oil adjuvant vaccine of the present invention is prepared, the W/O emulsion prepared in Step 1 preferably contains an oil component (A) in a proportion of 30 - 90 wt%, preferably 40 - 80 wt%, an emulsifier (B) in a proportion of 1 - 20 wt%, preferably 2 - 15 wt%, and an aqueous component (C) containing an antigen, in a proportion of 5 - 75 wt%, preferably 15 - 60 wt%, all relative to the W/O emulsion. The emulsifier (D) is used in Step 2 in a proportion of 0.1 - 20 wt%, preferably 0.5 - 10 wt%, of the total of the emulsifier (D), the aqueous component (E) and the polyethylene glycol derivative.

The mixing ratio of the W/O emulsion (prepared in Step 1) and a liquid containing an emulsifier (D) and an

aqueous component (E) in Step 2 is determined as appropriate depending on the objective or selected antigen kind. The weight ratio preferably W/O emulsion: a liquid containing an emulsifier (D) and an aqueous component (E) = 2:1 - 1:10, preferably 2:1 - 1:5.

According to the present invention, a polyethylene glycol derivative is added to the outer aqueous phase by partly or entirely adding the above-mentioned polyethylene glycol derivative of the formula (I), which has a molecular weight of 400 - 20,000, in advance to the above-mentioned liquid containing an emulsifier (D) and an aqueous component (E). Alternatively, a W/O/W type oil adjuvant is prepared and the polyethylene glycol derivative separately dissolved in water, physiological saline or buffer is added. The amount of the polyethylene glycol derivative to be added is as mentioned above.

When an oil adjuvant vaccine containing plural kinds of antigens is prepared, W/O emulsions containing each kind of antigen in the inner aqueous phase are prepared, mixed, and dispersed in a liquid containing an emulsifier (D) and an aqueous component (E), as shown later. Alternatively, W/O/W type oil adjuvants for any number of antigens are prepared and mixed, as mentioned later.

The oil adjuvant vaccine of the present invention as a pharmaceutical product can contain various additives that are not directly involved in the adjuvant activity, such as buffer agent, antiseptics, osmotic pressure controlling agent and the like.

For the preparation of the oil adjuvant vaccine of the present invention, any apparatus capable of emulsifying can be used both for the preparation of a W/O emulsion containing an antigen in the inner aqueous phase (Step 1) and for dispersing or emulsifying the W/O emulsion in a liquid containing an emulsifier (D) and an aqueous component (E) (Step 2), a typical emulsifying apparatus (e.g., homomixer, homogenizer, CLEARMIX (manufactured by M TECHNIQUE) and the like), a membrane emulsifying apparatus using a porous membrane, a high pressure jet emulsifying apparatus and the like can be used. Where necessary, the W/O emulsion constituting the oil adjuvant vaccine of the present invention is passed through a membrane filter to make the particle size below a certain level.

In the preparation of a vaccine, moreover, each component to be used may be subjected to a sterilization treatment in advance, depending on the property thereof, such as sterilization by heating and sterilization by filtering.

When the oil adjuvant vaccine prepared by the present invention is applied to a body, a suitable administration route is employed, such as subcutaneous, intramuscular and intraperitoneal injections, and administration through mucosal membrane of nasal cavity and intestinal lumen. The dose and administration frequency are to be determined appropriately to achieve a desired immunological effect according to a method typically employed in the pertinent field.

The present invention is explained in detail by referring to illustrative examples.

In the following Examples, polyethylene glycol (hereinafter to be also referred to as PEG or Macrogol) was used as a polyethylene glycol derivative.

**[preparation of oil adjuvant vaccine]**

The oil adjuvant vaccine used in each of the following Examples was prepared by the following method using CLEARMIX CLM-0.8S (M TECHNIQUE). Emulsification was performed at room temperature and cooling water was used as necessary. Each component in the following compositions was subjected to a sterilization treatment by a suitable sterilization method. Each operation of stirring, emulsifying etc. was performed under a sterile environment. In Examples, "parts" means "parts by weight".

In the following preparations of oil adjuvant vaccines, the "outer aqueous phase" means a liquid to be mixed with a W/O emulsion for the preparation of a W/O/W type oil adjuvant vaccine.

**<<Example 1: preparation of vaccine 1>>**

In accordance with the composition of W/O-1 in Table 1, each component other than the antigen suspension was weighed and placed in a beaker. A polyoxyethylene hydrogenated castor oil was heated to 50°C and dissolved before use. An aqueous solution of sodium glutamate and sorbitol was mixed with sorbitan sesquioleate at a weight ratio of 1/1, stirred and added. The remaining sorbitan sesquioleate was added as it was. Thereto was added gradually the antigen suspension with stirring and mixed by stirring at 12,000 rpm for 5 min at normal temperature in CLEARMIX CLM-0.8S (M TECHNIQUE) to give a W/O type emulsion (W/O-1).

According to the composition of the outer aqueous phase 1 in Table 3, each component was dissolved in phosphate buffered saline (PBS, pH 7.4) (outer aqueous phase 1). The above-mentioned W/O-1 (1 part) and the outer aqueous phase 1 (1 part) were weighed in a beaker and mixed using CLEARMIX CLM-0.8S at 9,000 rpm for 5 min to give an oil adjuvant vaccine 1. In the following Examples and Comparative Examples, the W/O emulsion and the outer aqueous phase were mixed under the same conditions as in Example 1 for vaccine preparation, unless particularly specified.

**<<Example 2: preparation of vaccine 2>>**

The W/O type emulsion (W/O-1, 1 part) prepared in Example 1 and the outer aqueous phase 2 (1 part) prepared according to the composition of the outer aqueous phase 2 in Table 3 were weighed and an oil adjuvant vaccine 2 was prepared.

**<<Example 3: preparation of vaccine 3>>**

According to the composition of the W/O-2 in Table 1, each component other than the antigen suspension was weighed in a beaker. The polyoxyethylene hydrogenated castor oil was heated to 50°C and dissolved before use. Japanese Pharmacopoeia Macrogol 6000 was homogeneously dissolved in advance in the antigen suspension. Under the same conditions as in Example 1, a W/O type emulsion was prepared (W/O-2).

Then, W/O-2 (3 parts) and the outer aqueous phase 3 (2 parts) prepared according to the composition of the outer aqueous phase 3 in Table 3 were weighed and an oil adjuvant vaccine 3 was prepared.

**<<Example 4: preparation of vaccine 4>>**

According to the composition of the outer aqueous phase 4 in Table 3, each component was weighed. The components were heated to about 60°C, thoroughly mixed, dispersed and cooled to room temperature for use as outer aqueous phase 4. Then, a W/O type emulsion (W/O-2, 1 part) prepared in Example 3 and outer aqueous phase 4 (3 parts) were weighed, and oil adjuvant vaccine 4 was prepared.

**<<Example 5: preparation of vaccine 5>>**

According to the composition of W/O-3 in Table 1, each component other than the antigen solution was weighed in a beaker. In the same manner as in Example 1, the antigen suspension was added gradually while stirring the mixture and a W/O type emulsion (W/O-3) was prepared in the same manner as in Example 1.

Then, this W/O type emulsion (W/O-3, 2 parts) and outer aqueous phase 2 (3 parts) were weighed in a beaker and an oil adjuvant vaccine 5 was prepared.

**<<Example 6: preparation of vaccine 6>>**

The W/O type emulsion (W/O-3, 2 parts) prepared in Example 5 and outer aqueous phase 5 (2 parts) prepared according to the composition of the outer aqueous phase 5 in Table 4 were weighed and mixed using CLEARMIX CLM-0.8S at 9,000 rpm for 5 min to give an emulsion. Japanese Pharmacopoeia Macrogol 6000 was dissolved in PBS to the concentration of 10 wt% and the resulting solution (1 part) was added. The mixture was stirred gently to give oil adjuvant vaccine 6.

**<<Example 7: preparation of vaccine 7>>**

According to the composition of W/O-4 in Table 2, each component other than the antigen suspension was weighed in a beaker. In the same manner as in Example 1 using CLEARMIX CLM-0.8S, a W/O type emulsion was prepared (W/O-4).

Then, W/O-4 (1 part) and outer aqueous phase 4 (1 part) prepared according to the composition of the outer aqueous phase 4 in Table 3 were weighed and an oil adjuvant vaccine 7 was prepared.

**<<Example 8: preparation of vaccine 8>>**

According to the composition of W/O-5 in Table 2, each component other than the antigen suspension was weighed in a beaker. The polyoxyethylene hydrogenated castor oil was heated to 60°C and dissolved before use. The antigen suspension was added gradually with stirring and a W/O type emulsion (W/O-5) was prepared in the same manner as in Example 1.

Then, W/O-5 (1 part) and outer aqueous phase 2 (1 part) were weighed and an oil adjuvant vaccine 8 was prepared.

**<<Example 9: preparation of vaccine 9>>**

According to the composition of W/O-6 in Table 2, each component other than the antigen suspension was

weighed in a beaker. Egg yolk lecithin was homogeneously dissolved in the oil phase and polyoxyethylene hydrogenated castor oil was heated to 60°C and dissolved before use. The antigen suspension was added gradually with stirring and a W/O type emulsion (W/O-6) was prepared in the same manner as in Example 1.

Then, W/O-6 (3 parts) and outer aqueous phase 3 (2 parts) were weighed and an oil adjuvant vaccine 9 was prepared.

**<<Comparative Example 1: preparation of vaccine 10>>**

The W/O-1 (1 part) prepared in Example 1 and outer aqueous phase 5 (1 part) shown in Table 4 were weighed and an oil adjuvant vaccine 10 was prepared.

**<<Comparative Example 2: preparation of vaccine 11>>**

The W/O-1 (1 part) prepared in Example 1 and outer aqueous phase 6 (1 part) shown in Table 4 were weighed and an oil adjuvant vaccine 11 was prepared.

**<<Comparative Example 3: preparation of vaccine 12>>**

The W/O-2 (3 parts) prepared in Example 3 and outer aqueous phase 7 (2 parts) shown in Table 4 were weighed and an oil adjuvant vaccine 12 was prepared.

**<<Comparative Example 4: preparation of vaccine 13>>**

The W/O-3 (2 parts) prepared in Example 5 and outer aqueous phase 5 (3 parts) shown in Table 4 were weighed and an oil adjuvant vaccine 13 was prepared.

**<<Comparative Example 5: preparation of vaccine 14>>**

The W/O-4 (1 part) prepared in Example 7 and outer aqueous phase 8 (1 part) shown in Table 4 were weighed and an oil adjuvant vaccine 14 was prepared.

**<<Comparative Example 6: preparation of vaccine 15>>**

The W/O-5 (1 part) prepared in Example 8 and outer aqueous phase 6 (1 part) shown in Table 4 were weighed and an oil adjuvant vaccine 15 was prepared.

**<<Comparative Example 7: preparation of vaccine 16>>**

The W/O-6 (3 parts) prepared in Example 9 and outer aqueous phase 7 (2 parts) shown in Table 4 were weighed and an oil adjuvant vaccine 16 was prepared.

The compositions of the W/O emulsions used for the vaccines of Examples and Comparative Examples are shown in Tables 1 and 2, the compositions of the outer aqueous phases are shown in Tables 3 and 4, and the mixing ratios of the W/O emulsion and the outer aqueous phase of the vaccines are shown in Table 5.

Table 1

| **Composition of W/O emulsion - 1** | | | |
|---|---|---|---|
| Components added | Mixing ratio (wt%) | | |
| compound | W/O-1 | W/O-2 | W/O-3 |
| ethyl oleate | 40 | - | - |
| squalene | - | 55 | 20 |
| purified soybean oil | - | - | 15 |
| sorbitan sesquioleate | 10 | 10 | 10 |
| polyoxyethylene (20) hydrogenated castor oil*1) | - | - | 5 |

*1) Figures in the parentheses show average numbers of moles of ethylene oxide added.

Table 1 (continued)

| Composition of W/O emulsion - 1 | | | |
|---|---|---|---|
| Components added | Mixing ratio (wt%) | | |
| compound | W/O-1 | W/O-2 | W/O-3 |
| polyoxyethylene (40) hydrogenated castor oil*1) | 5 | 3 | - |
| polyoxyethylene (20) sorbitan monooleate*1) | - | 2 | - |
| aqueous solution*2) of sodium glutamate and sorbitol | 5 | - | - |
| antigen suspension | 40 | 29 | 50 |
| Japanese Pharmacopoeia Macrogol 6000*3) | - | 1 | - |
| Total | 100 | 100 | 100 |

*1) Figures in the parentheses show average numbers of moles of ethylene oxide added.
*2) Aqueous solution (100 mL) prepared by dissolving sodium glutamate monohydrate (25 g) and sorbitol (10 g) in distilled water.
*3) Macrogol was dissolved in advance in the antigen suspension.

Table 2

| Composition of W/O emulsion - 2 | | | |
|---|---|---|---|
| Components added | Mixing ratio (wt%) | | |
| compound | W/O-4 | W/O-5 | W/O-6 |
| oleyl oleate | 40 | - | 30 |
| squalene | - | - | 20 |
| isopropyl myristate | - | 60 | - |
| purified yolk lecithin | - | - | 2 |
| glycerol monooleate | 15 | 10 | 8 |
| polyoxyethylene(10)hydrogenated castor oil*1) | 5 | 5 | - |
| polyoxyethylene(60)hydrogenated castor oil*1) | - | 5 | 5 |
| antigen suspension | 40 | 20 | 35 |
| Total | 100 | 100 | 100 |

*1) Figures in the parentheses show average numbers of moles of ethylene oxide added.

Table 3

| Composition of outer aqueous phase of oil adjuvant vaccine - 1 | | | | |
|---|---|---|---|---|
| Components added | Mixing ratio (%) | | | |
| compound | outer aqueous phase 1 | outer aqueous phase 2 | outer aqueous phase 3 | outer aqueous phase 4 |
| polyoxyethylene (60)hydrogenated castor oil*1) | 2 | - | - | 2 |
| polyoxyethylene (20)sorbitan monooleate*1) | - | - | 2 | - |

*1) Figures in the parentheses show average numbers of moles of ethylene oxide added.

Table 3 (continued)

| Composition of outer aqueous phase of oil adjuvant vaccine - 1 | | | | |
|---|---|---|---|---|
| Components added | Mixing ratio (%) | | | |
| compound | outer aqueous phase 1 | outer aqueous phase 2 | outer aqueous phase 3 | outer aqueous phase 4 |
| polyoxyethylene (196) polyoxypropylene (67)glycol*1) | 1 | 3 | - | - |
| purified yolk lecithin | - | - | - | 2 |
| Macrogol 1000*2) | - | - | - | 1 |
| Japanese Pharmacopoeia Macrogol 4000 | - | 4 | 1 | - |
| Japanese Pharmacopoeia Macrogol 6000 | 3 | - | 1 | - |
| phosphate buffered saline (PBS) | 94 | 93 | 96 | 95 |
| Total | 100 | 100 | 100 | 100 |

*1) Figures in the parentheses show average numbers of moles of ethylene oxide added.
*2) Japanese Pharmaceutical Excipients acceptable product

Table 4

| Composition of outer aqueous phase of oil adjuvant vaccine - 2 | | | | |
|---|---|---|---|---|
| Components added | Mixing ratio (%) | | | |
| compound | outer aqueous phase 5 | outer aqueous phase 6 | outer aqueous phase 7 | outer aqueous phase 8 |
| polyoxyethylene (60)hydrogenated castor oil*1) | 2 | - | - | 2 |
| polyoxyethylene (20)sorbitan monooleate*1) | - | - | 2 | - |
| polyoxyethylene (196) polyoxypropylene (67)glycol*1) | 1 | 3 | - | - |
| purified yolk lecithin | - | - | - | 2 |
| phosphate buffered saline (PBS) | 97 | 97 | 98 | 96 |

*1) Figures in the parentheses show average numbers of moles of ethylene oxide added.

Table 4 (continued)

| Composition of outer aqueous phase of oil adjuvant vaccine - 2 | | | | |
|---|---|---|---|---|
| Components added | Mixing ratio (%) | | | |
| compound | outer aqueous phase 5 | outer aqueous phase 6 | outer aqueous phase 7 | outer aqueous phase 8 |
| Total | 100 | 100 | 100 | 100 |

Table 5

| W/O emulsion and outer aqueous phase used for each vaccine preparation and amounts added thereof | | | |
|---|---|---|---|
| Vaccine No. | | W/O emulsion | Outer aqueous phase |
| Example | Vaccine 1 | W/O-1 (1 part) | outer aqueous phase 1 (1 part) |
| | Vaccine 2 | W/O-1 (1 part) | Outer aqueous phase 2 (1 part) |
| | Vaccine 3 | W/O-2 (3 parts) | outer aqueous phase 3 (2 parts) |
| | Vaccine 4 | W/O-2 (1 part) | outer aqueous phase 4 (3 parts) |
| | Vaccine 5 | W/O-3 (2 parts) | outer aqueous phase 2 (3 parts) |
| | Vaccine 6 | W/O-3 (2 parts) | outer aqueous phase 5 (2 parts)<br>Macrogol solution (1 part)*1) |
| | Vaccine 7 | W/O-4 (1 part) | outer aqueous phase 4 (1 part) |
| | Vaccine 8 | W/O-5 (1 part) | outer aqueous phase 2 (1 part |
| | Vaccine 9 | W/O-6 (3 parts) | outer aqueous phase 3 (2 parts) |
| Comparative Example | Vaccine 10 | W/O-1 (1 part) | outer aqueous phase 5 (1 part) |
| | Vaccine 11 | W/O-1 (1 part) | outer aqueous phase 6 (1 part) |
| | Vaccine 12 | W/O-2 (3 parts) | outer aqueous phase 7 (2 parts) |
| | Vaccine 13 | W/O-3 (2 parts) | outer aqueous phase 5 (3 parts) |
| | Vaccine 14 | W/O-4 (1 part) | outer aqueous phase 8 (1 part) |
| | Vaccine 15 | W/O-5 (1 part) | outer aqueous phase 6 (1 part) |
| | Vaccine 16 | W/O-6 (3 parts) | outer aqueous phase 7 (2 parts) |

*1) See Example 6 in the Specification for the detail of the preparation method.

[Immunity test]

The oil adjuvant vaccines containing various antigens were subjected to immunity tests. The symbols of the vaccines used in respective Experimental Examples correspond to those used in the aforementioned Examples and Comparative Examples.

**<<Experimental Example 1: Swine *Erysipelothrix rhusiopathiae* (Er) inactivated vaccine>>**

The culture of *Erysipelothrix rhusiopathiae* SE-9 strain (AMERICAN TYPE CULTURE COLLECTION) in medium containing 5% bovine serum and brain heart infusion broth (BHIB) was inactivated with formalin to give an antigen. Using the antigen, vaccines 1, 3, 5, 7, 10, 12 and 14 were prepared. For preparation, the antigen concentration of each vaccine was appropriately adjusted to $5.0\times 10^8$ CFU/ml with phosphate buffered saline (PBS). Placebo containing PBS instead of the antigen solution was prepared for each vaccine. Each vaccine was diluted with the corresponding placebo to give a 5-fold serial dilution. As the control, Freund's incomplete adjuvant (IFA), an adjuvant (IFA-PEG) wherein PEG 1000 was added to the inner aqueous phase of IFA in a proportion of 5% of the inner aqueous phase

and a vaccine containing an aluminum hydroxide gel as an adjuvant were prepared.

Each vaccine (or diluted vaccine) was inoculated (0.25 ml/mouse) subcutaneously to the inner thigh of 5-week-old Clean mouse (5 mice per group). After 3 weeks from the immunization, Er Fujisawa strain [Japanese Association of Veterinary Biologics (JAVB)] was injected subcutaneously ($2.2\times10^6$ CFU/0.1 ml/mouse) into the inner thigh of each mouse. After the injection, the survival of the mice was monitored for 10 days, and 50% survival vaccine dilution multiple as expressed in $PD_{50}$ was calculated. After 2 weeks from the immunization, an undiluted vaccine and a 5-fold diluted vaccine were inoculated to the mice (10 mice for each formulation) and the injection site was examined by palpation to determine the degree of induration. The degree of induration was scored in 5 levels (0: none, 1:light - 4:severe) and an average was calculated for each formulation.

The W/O/W type oil adjuvant vaccines prepared at this time were measured for the average particle size by a laser diffraction particle size analyzer SALD-2100 (SHIMADZU CORPORATION).

The results of effectiveness ($PD_{50}$) are summarized in Table 6.

**Table 6: Swine erysipelas - mouse immunization test results (Experimental Example 1)**

| | Test results (survived mice/mice tested) | | | | | | | $PD_{50}$ |
|---|---|---|---|---|---|---|---|---|
| | undiluted vaccine | × 5 | × 25 | × 125 | × 625 | × 3125 | × 15625 | (fold) |
| [immunization group] | | | | | | | | |
| Vaccine 1 | 5/5 | 5/5 | 5/5 | 5/5 | 4/5 | 3/5 | 0/5 | 2660.4 |
| Vaccine 3 | 5/5 | 5/5 | 5/5 | 5/5 | 3/5 | 2/5 | 0/5 | 1397.5 |
| Vaccine 5 | 5/5 | 5/5 | 5/5 | 5/5 | 4/5 | 2/5 | 0/5 | 1928.2 |
| Vaccine 7 | 5/5 | 5/5 | 5/5 | 5/5 | 4/5 | 1/5 | 0/5 | 1397.5 |
| [immunization group for comparison] | | | | | | | | |
| Vaccine 10 | 5/5 | 5/5 | 5/5 | 5/5 | 2/5 | 0/5 | 0/5 | 532.1 |
| Vaccine 12 | 5/5 | 5/5 | 5/5 | 3/5 | 2/5 | 1/5 | 0/5 | 385.7 |
| Vaccine 14 | 5/5 | 5/5 | 5/5 | 3/5 | 2/5 | 0/5 | 0/5 | 279.5 |
| IFA | 5/5 | 5/5 | 5/5 | 5/5 | 5/5 | 3/5 | 0/5 | 3670.7 |
| IFA-PEG | 5/5 | 5/5 | 5/5 | 5/5 | 4/5 | 1/5 | 0/5 | 1397.5 |
| aluminum gel | 5/5 | 5/5 | 4/5 | 2/5 | 0/5 | 0/5 | 0/5 | 77.1 |

The results are shown in the number of the survived mice inoculated with a diluted vaccine (or an undiluted vaccine) and $PD_{50}$ values thereof. As used herein, a greater $PD_{50}$ shows higher effectiveness. Of the immunization groups, the vaccine 1, 3, 5, 7 inoculation group showed a $PD_{50}$ of not less than 1300, exhibiting superior effectiveness comparable to IFA. In contrast, the immunization group for comparison (administered with vaccine 10, 12, 14 and aluminum gel adjuvant) showed $PD_{50}$ values of 532.1, 385.7, 279.5 and 77.1. It is evident that the vaccine prepared by the present invention showed significantly high $PD_{50}$ values. These results have established that the vaccine prepared by the present invention is highly effective.

Then, the injection site was examined by palpation to determine the degree of induration. The average particle size of the W/O/W type oil adjuvant vaccine is shown in Table 7 along with the degree of induration.

Table 7: Swine erysipelas - topical response of injection site in mouse (Experimental Example 1)

| | Topical response (average) of injection site | Average particle size (μm) |
|---|---|---|
| [immunization group] | | |
| Vaccine 1 | 0.1 | 6.8 |
| Vaccine 3 | 0.2 | 8.1 |
| Vaccine 5 | 0 | 7.2 |
| Vaccine 7 | 0.2 | 7.9 |
| [immunization group for comparison] | | |
| Vaccine 10 | 0.3 | 7.5 |
| Vaccine 12 | 0.2 | 8.5 |
| Vaccine 14 | 0.8 | 8.8 |
| IFA | 3.8 | - |
| IFA-PEG | 3.2 | - |
| aluminum gel | 0 | - |

score of topical response at injection site (0:none, 1: light - 4: severe)

The immunization group scarcely showed reaction at the injection site, thereby demonstrating high safety of the injection, and the average particle size was not more than 7.2 - 8.1 or 20 μm. In contrast, the immunization group for comparison showed an average particle size of the W/O/W type oil adjuvant vaccine of almost the same level, and the response at the injection site of the immunization group for comparison except IFA and IFA-PEG was small. However, the mice in the IFA and IFA-PEG injection groups developed severe induration, making the reaction score extremely high, wherein about 3/4 or more of the mice inoculated with IFA or IFA-PEG suffered from disturbance in gait.

The foregoing results establish that the oil adjuvant vaccine of the present invention shows dramatically superior effectiveness as well as extremely light response at the injection site as with an aluminum gel vaccine. The inventive vaccine is highly safe.

**<<Experimental Example 2: Swine Mycoplasma inactivated vaccine>>**

Vaccines 2, 4, 6, 11 and 13 were prepared from *Mycoplasma hyopneumoniae* 92-28 strain (strain isolated from field swine; Juridical Foundation, The Chemo-Sero-Therapeutic Research Institute) cultured in BHL medium supplemented with swine serum and inactivated with formalin. For preparation, the antigen content of each vaccine was adjusted with phosphate buffered saline (PBS) to the antigen concentration of $8.0\times10^8$ CCU/ml. A vaccine (vaccine AL) using an aluminum hydroxide gel (ALHYDROGEL 85, Superfos, aluminum conversion 9 mg/ml) as an adjuvant, and a vaccine (vaccine AL-PEG) obtained by adding Japanese Pharmacopoeia Macrogol 4000 to an aluminum hydroxide gel to the final concentration of 4% were adjusted to have the same antigen concentration.

Each vaccine (2 ml/swine) was inoculated twice to SPF (Specific Pathogen Free) swine (8 swine/group, 4-week-old at the time of first immunization) into the cervical muscle at 2 week intervals. After one week from the second immunization, lung homogenate ($3\times10^6$ CCU/6 ml/swine) derived from swine infected with *Mycoplasma hyopneumoniae* 92-28 strain (strain isolated from field swine; Juridical Foundation, The Chemo-Sero-Therapeutic Research Institute) was administered into the trachea. After four weeks from the administration, the swine were subjected to autopsy, and lung lesion was observed. Respective lung lesions were scored, and a decrease (%) in the lesion by each vaccine relative to the lesion of the non-immunized control swine was calculated.

The results are shown in Table 8.

Table 8

| Swine *Mycoplasma* infection - mouse immunization test results (Experimental Example 2) | | | | | | | | |
|---|---|---|---|---|---|---|---|---|
| | Immunization group | | | Immunization group for comparison | | | | |
| | Vaccine 2 | Vaccine 4 | Vaccine 6 | Vaccine 11 | Vaccine 13 | Vaccine AL | Vaccine AL-PEG | Non-immunization control |
| Lesion score (each individual) | 0 | 0 | 0 | 15.2 | 16.4 | 23.6 | 50.2 | 5.5 |
| | 1.5 | 2.0 | 0 | 0.5 | 5.5 | 15.2 | 16.5 | 11.8 |
| | 0.5 | 9.6 | 3.6 | 9.1 | 22.0 | 5.5 | 22.1 | 14.5 |
| | 16.2 | 30.2 | 5.8 | 4.5 | 4.58 | 19.6 | 25.9 | 32.7 |
| | 2.6 | 5.5 | 18.6 | 14.2 | 19.5 | 56.3 | 30.6 | 15.9 |
| | 9.2 | 16.3 | 20.6 | 23.0 | 23.3 | 12.4 | 11.1 | 40.2 |
| | 3.5 | 1.5 | 9.6 | 38.6 | 36.2 | 28.4 | 9.1 | 25.5 |
| | 19.6 | 10.5 | 10.2 | 10.2 | 14.4 | 30 | 30 | 45.5 |
| (Average) | 6.6 | 9.5 | 8.6 | 14.4 | 17.7 | 23.9 | 24.4 | 24.0 |
| Decrease (%) in lesion | 72.3 | 60.5 | 64.3 | 39.8 | 26.0 | 0.3 | -2.0 | - |

The immunization groups inoculated with vaccines 2, 4 and 6 showed decrease in the lesion by 60% or above, establishing superior effectiveness. The vaccine 6, to which PEG was added after secondary emulsification, showed almost the same effect as other immunization groups. In contrast, the immunization groups for comparison administered with vaccines 11 and 13 showed a decrease in the lesion by 39.8% and 26.0%, respectively, and the aluminum gel vaccine did not at all show a lesion-decreasing effect by the vaccine, irrespective of the presence or otherwise of PEG addition.

The foregoing effects establish that the oil adjuvant vaccine of the present invention shows superior effects even when Mycoplasma is used as an antigen. In addition, the results establish that the addition of PEG to the outer aqueous phase is effective whether before or after the secondary emulsification.

[Measurement of viscosity of emulsion]

The prepared W/O/W type oil adjuvant vaccines were measured for viscosity. The swine *Erysipelothrix rhusiopahtiae* vaccine (vaccines 1, 3, 5, 7, 10, 12, 14) prepared in the above Experimental Example 1, as well as respective vaccines prepared according to the formulations of vaccines 2, 4, 6, 8, 9, 11, 13, 15 and 16 and using the swine *Erysipelothrix rhusiopahtiae* as an antigen were subjected to the measurement, so that the comparison can be made under the same formulation of antigen suspension. The viscosity was measured one week from the vaccine preparation, using an E type rotary viscometer (TOKI SANGYO CO., LTD., RE105H type) at 25°C. The results are shown in Table 9.

Table 9

| Viscosity of vaccine preparation | | |
|---|---|---|
| Vaccine No. | | Viscosity (mPa·s, 25°C) |
| Example | Vaccine 1 | 33 |
| | Vaccine 2 | 28 |
| | Vaccine 3 | 42 |
| | Vaccine 4 | 35 |
| | Vaccine 5 | 31 |
| | Vaccine 6 | 32 |
| | Vaccine 7 | 38 |
| | Vaccine 8 | 45 |
| | Vaccine 9 | 29 |
| Comparative Example | Vaccine 10 | 153 |
| | Vaccine 11 | 161 |
| | Vaccine 12 | 206 |
| | Vaccine 13 | 187 |
| | Vaccine 14 | 470 |
| | Vaccine 15 | 396 |
| | Vaccine 16 | 315 |

As shown in Table 9, the vaccines of Examples all showed a viscosity at 25°C of not more than 50 mPa·s. The viscosity was very low and it facilitated injection. In contrast, the vaccines of Comparative Examples had a viscosity of not less than 150 mPa·s, necessitating a considerable force and time for injection.

The above results suggest that the W/O/W type oil adjuvant vaccine of the present invention can have significantly reduced viscosity by containing a polyethylene glycol derivative in the outer aqueous phase, thus indicating that the administration of the inventive vaccine can save labor to a great extent.

[Measurement of inclusion rate of emulsion]

The prepared W/O/W type oil adjuvant vaccines were measured for inclusion rate. Swine Mycoplasma vaccine (vaccines 2, 4, 6, 11, 13) used in Experimental Example 2 above, as well as respective vaccines prepared according to the formulations of vaccines 1, 3, 5, 7, 8, 9, 10, 12, 14, 15 and 16 and using said Mycoplasma as an antigen were subjected to the measurement, so that the comparison can be made under the same formulation of antigen suspension. The prepared vaccines were diluted with PBS, centrifuged to separate the emulsion part from the outer aqueous phase. These were harvested and the protein amount in the outer aqueous phase was measured. The protein amount in the outer aqueous phase was subtracted from that in the antigen suspension (constituting the inner aqueous phase) added during the preparation of W/O emulsion, to determine the amount of the protein retained in the inner aqueous phase, based on which the inclusion rate was calculated from the following formula.

Inclusion rate (%)= [(amount of protein in added antigen solution)

- (amount of protein in outer aqueous phase)] / amount of

protein in added antigen suspension

The vaccines were stored at 4°C after preparation, and the inclusion rate was measured on the next day of the vaccine preparation and 3 months later.

The results of the inclusion rate are shown in Table 10.

Table 10

| Inclusion rate of prepared vaccine | | | |
|---|---|---|---|
| | | Inclusion rate (%) | |
| | | Next day of preparation | 3 months after preparation |
| Example | Vaccine 1 | 98 | 97 |
| | Vaccine 2 | 98 | 98 |
| | Vaccine 3 | 93 | 90 |
| | Vaccine 4 | 94 | 92 |
| | Vaccine 5 | 93 | 93 |
| | Vaccine 6 | 92 | 90 |
| | Vaccine 7 | 94 | 92 |
| | Vaccine 8 | 97 | 97 |
| | Vaccine 9 | 92 | 89 |
| Comparative Example | Vaccine 10 | 97 | 95 |
| | Vaccine 11 | 98 | 98 |
| | Vaccine 12 | 81 | 71 |
| | Vaccine 13 | 91 | 90 |
| | Vaccine 14 | 96 | 96 |
| | Vaccine 15 | 94 | 94 |
| | Vaccine 16 | 87 | 85 |

In both the Examples and Comparative Examples, high inclusion rate of not less than 70% was observed the next day of preparation and 3 months from the preparation, thus demonstrating superior stability. Particularly, the vaccines of Examples showed extremely low viscosity as shown in Table 9 and superior protein inclusion rate.

The foregoing results establish that the W/O/W type oil adjuvant vaccine of the present invention having dramatically low viscosity has high stability.

As is evident from the results shown in Table 6 - Table 10, the oil adjuvant vaccine of the present invention exhibits well-balanced superior effectiveness characteristic of the W/O type oil adjuvant and superior safety characteristic of the O/W type oil adjuvant. The constitution of the present invention, where a specific polyethylene glycol derivative is contained in the outer aqueous phase of the W/O/W type oil adjuvant vaccine, enables preparation of a useful oil adjuvant vaccine which is free of an influence from the components contained in the inner aqueous phase and which is superior in preparation stability and workability to allow a person to give an injection easily due to the lowered viscosity.

The W/O/W type oil adjuvant vaccine of the present invention affords a high adjuvant effect and shows superior preparation stability and fewer side effects such as topical response.

## Claims

**1.** A W/O/W type oil adjuvant vaccine comprising an outer aqueous phase comprising 0.5- 20 wt% of a polyethylene glycol derivative having a molecular weight of 400 - 20,000, which is represented by the following formula (I)

$$R^1-O-(CH_2-CH_2-O)_n-R^2 \qquad (I)$$

wherein $R^1$ and $R^2$ may be the same or different and each is a hydrogen atom or alkyl having 1 to 4 carbon atoms

and n is a polymerization degree, and an inner aqueous phase comprising a biologically acceptable and effective amount of an antigen.

2. The oil adjuvant vaccine of claim 1, wherein the polyethylene glycol derivative of the formula (I) has a molecular weight of 1,000 - 10,000.

3. The oil adjuvant vaccine of claim 1, wherein the outer aqueous phase comprises 1 - 10 wt% of the polyethylene glycol derivative of the formula (I).

4. The oil adjuvant vaccine of claim 1, which is a W/O/W type oil adjuvant vaccine prepared by the steps of

(a) preparing a W/O emulsion comprising an oil component (A) which becomes liquid at a temperature in the range of 15-25°C, an emulsifier (B) and an aqueous component (C) comprising a biologically acceptable and effective amount of an antigen, and
(b) dispersing or emulsifying the W/O emulsion in a liquid comprising an emulsifier (D) and an aqueous component (E),

wherein the liquid comprises 0.5 - 20 wt% of a polyethylene glycol derivative having a molecular weight of 400 - 20,000, which is represented by the formula (I).

5. The oil adjuvant vaccine of claim 1, which is a W/O/W type oil adjuvant vaccine prepared by the steps of

(a) preparing a W/O emulsion comprising an oil component (A) which becomes liquid at a temperature in the range of 15-25°C, an emulsifier (B) and an aqueous component (C) comprising a biologically acceptable and effective amount of an antigen,
(b) dispersing or emulsifying the W/O emulsion in a liquid comprising an emulsifier (D) and an aqueous component (E), and
(c) adding a polyethylene glycol derivative having a molecular weight of 400 - 20,000, which is represented by the formula (I), to the outer aqueous phase to a concentration of 0.5 - 20 wt%.

6. The oil adjuvant vaccine of claim 4 or claim 5, wherein the oil component (A), which becomes liquid at a temperature in the range of 15-25°C, comprises a fatty acid ester or squalene or a fatty acid ester and squalene in a proportion of not less than 20 wt% of an oil phase.

7. The oil adjuvant vaccine of claim 4 or claim 5, wherein the emulsifier (B) has an HLB of less than 10.

8. The oil adjuvant vaccine of claim 7, wherein the emulsifier (B) comprises at least one member selected from the group consisting of a partial ester of polyhydric alcohol and a fatty acid, and a non-ionic surfactant having a polyoxyethylene chain.

9. The oil adjuvant vaccine of claim 4 or claim 5, wherein the emulsifier (D) has an HLB of not less than 10.

10. The oil adjuvant vaccine of claim 9, wherein the emulsifier (D) comprises a non-ionic surfactant having a polyoxyethylene chain.

**Patentansprüche**

1. Öladjuvansimpfstoff des w/o/w-Typs mit einer äußeren wässrigen Phase, die 0,5 bis 20 Gew.-% eines Polyethylenglycolderivats mit einem Molekulargewicht von 400 bis 20 000 umfasst, das durch die folgende Formel (I) dargestellt wird:

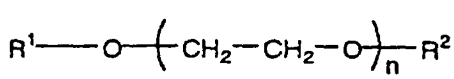
(I)

wobei $R^1$ und $R^2$ gleich oder verschieden sein können und jeweils ein Wasserstoffatom oder Alkyl mit 1 bis 4 Kohlenstoffatomen sind und n ein Polymerisationsgrad ist, und einer inneren wässrigen Phase, die eine biologisch

annehmbare und effektive Menge eines Antigens umfasst.

2. Öladjuvansimpfstoff gemäß Anspruch 1, wobei das Polyethylenglycolderivat der Formel (I) ein Molekulargewicht von 1000 bis 10 000 hat.

3. Öladjuvansimpfstoff gemäß Anspruch 1, wobei die äußere wässrige Phase 1 bis 10 Gew.-% des Polyethylenglycolderivats der Formel (I) umfasst.

4. Öladjuvansimpfstoff gemäß Anspruch 1, bei dem es sich um einen Öladjuvansimpfstoff des w/o/w-Typs handelt, der durch die folgenden Schritte hergestellt wird:

(a) Herstellen einer w/o-Emulsion mit einer Ölkomponente (A), die bei einer Temperatur im Bereich von 15 bis 25°C flüssig wird, einem Emulgator (B) und einer wässrigen Komponente (C), die eine biologisch annehmbare und effektive Menge eines Antigens umfasst; und

(b) Dispergieren oder Emulgieren der w/o-Emulsion in einer Flüssigkeit, die einen Emulgator (D) und eine wässrige Komponente (E) umfasst, wobei die Flüssigkeit 0,5 bis 20 Gew.-% eines Polyethylenglycolderivats mit einem Molekulargewicht von 400 bis 20 000 umfasst, das durch die Formel (I) dargestellt wird.

5. Öladjuvansimpfstoff gemäß Anspruch 1, bei dem es sich um einen Öladjuvansimpfstoff des w/o/w-Typs handelt, der durch die folgenden Schritte hergestellt wird:

(a) Herstellen einer w/o-Emulsion mit einer Ölkomponente (A), die bei einer Temperatur im Bereich von 15 bis 25°C flüssig wird, einem Emulgator (B) und einer wässrigen Komponente (C), die eine biologisch annehmbare und effektive Menge eines Antigens umfasst; und

(b) Dispergieren oder Emulgieren der w/o-Emulsion in einer Flüssigkeit, die einen Emulgator (D) und eine wässrige Komponente (E) umfasst; und

(c) Hinzufügen eines Polyethylenglycolderivats mit einem Molekulargewicht von 400 bis 20 000, das durch die Formel (I) dargestellt wird, zu der äußeren wässrigen Phase in einer Konzentration von 0,5 bis 20 Gew.-%.

6. Öladjuvansimpfstoff gemäß Anspruch 4 oder 5, wobei die Ölkomponente (A), die bei einer Temperatur im Bereich von 15 bis 25 °C flüssig wird, einen Fettsäureester oder Squalen oder sowohl einen Fettsäureester als auch Squalen in einem Anteil von nicht weniger als 20 Gew.-% der Ölphase umfasst.

7. Öladjuvansimpfstoff gemäß Anspruch 4 oder 5, wobei der Emulgator (B) ein HLB von weniger als 10 hat.

8. Öladjuvansimpfstoff gemäß Anspruch 7, wobei der Emulgator (B) wenigstens einen Vertreter umfasst, der aus der Gruppe ausgewählt ist, die aus einem partiellen Ester eines mehrwertigen Alkohols und einer Fettsäure sowie einem nichtionischen Tensid mit einer Polyoxyethylenkette besteht.

9. Öladjuvansimpfstoff gemäß Anspruch 4 oder 5, wobei der Emulgator (D) ein HLB von nicht weniger als 10 hat.

10. Öladjuvansimpfstoff gemäß Anspruch 9, wobei der Emulgator (D) ein nichtionisches Tensid mit einer Polyoxyethylenkette umfasst.

## Revendications

1. Vaccin à adjuvant à l'huile de type eau dans l'huile dans l'eau comprenant une phase aqueuse externe comprenant 0,5 - 20% en poids d'un dérivé polyéthylèneglycol ayant une masse moléculaire de 400 - 20 000, qui est représenté par la formule (1) suivante

$$R^1—O—(-CH_2—CH_2—O-)_n—R^2 \qquad (1)$$

dans laquelle $R^1$ et $R^2$ peuvent être identiques ou différents et chacun est un atome d'hydrogène ou un groupe

alkyle comportant 1 à 4 atomes de carbone, et n est un degré de polymérisation, et une phase aqueuse interne comprenant une quantité efficace et acceptable sur le plan biologique d'un antigène.

**2.** Vaccin à adjuvant à l'huile selon la revendication 1, dans lequel le dérivé polyéthylèneglycol de formule (I) a une masse moléculaire de 1 000 - 10 000.

**3.** Vaccin à adjuvant à l'huile selon la revendication 1, dans lequel la phase aqueuse externe comprend 1 - 10% en poids du dérivé polyéthylèneglycol de formule (I).

**4.** Vaccin à adjuvant à l'huile selon la revendication 1, qui est un vaccin à adjuvant à l'huile de type eau dans l'huile dans l'eau préparé par les étapes consistant à

(a) préparer une émulsion eau dans l'huile comprenant un constituant huileux (A) qui devient liquide à une température ambiante dans la gamme de 15-25°C, un émulsifiant (B) et un constituant aqueux (C) comprenant une quantité efficace et acceptable sur le plan biologique d'un antigène, et
(b) disperser ou émulsionner l'émulsion eau dans l'huile dans un liquide comprenant un émulsifiant (D) et un constituant aqueux (E),

dans lequel le liquide comprend 0,5 - 20% en poids d'un dérivé polyéthylèneglycol ayant une masse moléculaire de 400 - 20 000, qui est représenté par la formule (I).

**5.** Vaccin à adjuvant à l'huile selon la revendication 1, qui est un vaccin à adjuvant à l'huile de type eau dans l'huile dans l'eau préparé par les étapes consistant à

(a) préparer une émulsion eau dans l'huile comprenant un constituant huileux (A) qui devient liquide à une température ambiante dans la gamme de 15-25°C, un émulsifiant (B) et un constituant aqueux (C) comprenant une quantité efficace et acceptable sur le plan biologique d'un antigène,
(b) disperser ou émulsionner l'émulsion eau dans l'huile dans un liquide comprenant un émulsifiant (D) et un constituant aqueux (E), et
(c) ajouter un dérivé polyéthylèneglycol ayant une masse moléculaire de 400 - 20 000, qui est représenté par la formule (I), à la phase aqueuse externe jusqu'à une concentration de 0,5 - 20% en poids.

**6.** Vaccin à adjuvant à l'huile selon la revendication 4 ou la revendication 5, dans lequel le constituant huileux (A), qui devient liquide à une température ambiante dans la gamme de 15-25°C, comprend un ester d'acide gras ou un squalène, ou un ester d'acide gras et un squalène, dans une proportion non inférieure à 20% en poids d'une phase huileuse.

**7.** Vaccin à adjuvant à l'huile selon la revendication 4 ou la revendication 5, dans lequel l'émulsifiant (B) possède un rapport hydrolipophile inférieur à 10.

**8.** Vaccin à adjuvant à l'huile selon la revendication 7, dans lequel l'émulsifiant (B) comprend au moins un élément choisi dans le groupe constitué par un ester partiel de polyol et d'un acide gras et un tensioactif non ionique comportant une chaîne polyoxyéthylène.

**9.** Vaccin à adjuvant à l'huile selon la revendication 4 ou la revendication 5, dans lequel l'émulsifiant (D) possède un rapport hydrolipophile inférieur à 10.

**10.** Vaccin à adjuvant à l'huile selon la revendication 9, dans lequel l'émulsifiant (D) comprend un tensioactif non ionique comportant une chaîne polyoxyéthylène.